# EUROPEAN PATENT SPECIFICATION

(11) **EP 2 204 378 B1**
(45) Date of publication and mention of the grant of the patent: **14.11.2018**
(21) Application number: 08706521.5
(22) Date of filing: 13.02.2008
(51) Int. Cl.: C07K 7/06, C07K 7/08, C07K 14/435, A61K 38/08, A61K 38/10, A61K 38/17, A61P 35/00

(54) **A INHIBITING AGENT FOR INHIBITION OF ANGIOGENESIS, A METHOD FOR PREPARING THE AGENT, A METHOD FOR MODIFYING THE AGENT AND ITS USE FOR MANUFACTURING A MEDICAMENT FOR TREATING TUMOR**
INHIBIERENDES MITTEL ZUR INHIBIERUNG VON ANGIOGENESE, VERFAHREN ZUR HERSTELLUNG DES MITTELS, VERFAHREN ZUM MODIFIZIEREN DES MITTELS UND DESSEN VERWENDUNG ZUR HERSTELLUNG EINES MEDIKAMENTS ZUR BEHANDLUNG VON TUMOREN
AGENT INHIBITEUR POUR L'INHIBITION DE L'ANGIOGENÈSE, PROCÉDÉ DE PRÉPARATION DE L'AGENT, PROCÉDÉ POUR MODIFIER L'AGENT, ET SON UTILISATION POUR LA FABRICATION D'UN MÉDICAMENT DESTINÉ AU TRAITEMENT D'UNE TUMEUR

(30) Priority: 22.06.2007 CN 200710024565
(43) Date of publication of application: 07.07.2010
(73) Proprietor: Xu, Hanmei, Nanjing Jiangsu 210009 (CN)
(72) Inventor: Xu, Hanmei, Nanjing Jiangsu 210009 (CN)
(74) Representative: Kramer Barske Schmidtchen Patentanwälte PartG mbB
(86) International application number: PCT/CN2008/000346
(87) International publication number: WO 2009/000137

(56) References cited:
- WO-A1-00/18421
- WO-A2-2005/110489
- CN-A- 1 478 541
- CN-A- 1 699 408
- CN-A- 1 699 408
- CN-A- 1 876 186
- CN-A- 1 876 186
- US-B1- 6 825 167
- XU HAN-MEI ET AL: "An RGD-modified endostatin-derived synthetic peptide shows antitumor activity in vivo" BIOCONJUGATE CHEMISTRY, ACS, WASHINGTON, DC, US LNKD- DOI:10.1021/BC800132P, vol. 19, no. 10, 1 October 2008 (2008-10-01), pages 1980-1986, XP008098517 ISSN: 1043-1802 [retrieved on 2008-09-19]
- HERSEL U ET AL: "RGD modified polymers: biomaterials for stimulated cell adhesion and beyond" BIOMATERIALS, ELSEVIER SCIENCE PUBLISHERS BV., BARKING, GB LNKD- DOI:10.1016/S0142-9612(03)00343-0, vol. 24, no. 24, 1 November 2003 (2003-11-01), pages 4385-4415, XP004446082 ISSN: 0142-9612
- FISCHBACH ET AL: "Polymers for pro- and anti-angiogenic therapy" BIOMATERIALS, ELSEVIER SCIENCE PUBLISHERS BV., BARKING, GB LNKD- DOI:10.1016/J.BIOMATERIALS.2006.12.029, vol. 28, no. 12, 5 February 2007 (2007-02-05), pages 2069-2076, XP005876551 ISSN: 0142-9612
- TEMMING ET AL: "RGD-based strategies for selective delivery of therapeutics and imaging agents to the tumour vasculature" DRUG RESISTANCE UPDATES, CHURCHILL LIVINGSTONE, EDINBURGH, GB LNKD- DOI:10.1016/J.DRUP.2005.10.002, vol. 8, no. 6, 1 December 2005 (2005-12-01), pages 381-402, XP005328751 ISSN: 1368-7646
- WANG J. ET AL.: 'Progress of PEG Modified of Macromolecular Drug' CHINESE JOURNAL OF PHARMEUTICALS vol. 35, no. 11, 2004, pages 696 - 701, XP001525542
- HONEYCUTT L ET AL: "COMPARISON OF PHARMACOKINETIC PARAMETERS OF A POLYPEPTIDE, THE BOWMAN-BIRK PROTEASE INHIBITOR (BBI), AND ITS PALMITIC ACID CONJUGATE", PHARMACEUTICAL RESEARCH, KLUWER ACADEMIC PUBLISHERS, NEW YORK, NY, US, vol. 13, no. 9, 1 January 1996 (1996-01-01), pages 1373-1377, XP008029505, ISSN: 0724-8741, DOI: DOI:10.1023/A:1016078118033
- TSUNODA S ET AL: "Molecular design of polyvinylpyrrolidone-conjugated interleukin-6 for enhancement of in vivo thrombopoietic activity in mice", JOURNAL OF CONTROLLED RELEASE, ELSEVIER, AMSTERDAM, NL, vol. 68, no. 3, 3 September 2000 (2000-09-03), pages 335-341, XP004208504, ISSN: 0168-3659, DOI: DOI:10.1016/S0168-3659(00)00249-2
- GREGORIADIS G ET AL: "Polysialic acids: Potential in improving the stability and pharmacokinetics of proteins and other therapeutics", CMLS CELLULAR AND MOLECULAR LIFE SCIENCES, BIRKHAUSER VERLAG, HEIDELBERG, DE, vol. 57, no. 13-14, 1 December 2000 (2000-12-01), pages 1964-1969, XP002195628, ISSN: 1420-682X

## Description

### FIELD OF THE INVENTION

The present invention relates to biomedical technology field or protein polypeptide drug field, particularly relates to an antiangiogenesis agent, the production method thereof and its use in treating tumors.

### BACKGROUND OF THE INVENTION

Tumor angiogenesis inhibitor is new type of drug focused on in the treatment of tumors in recent years. Studies in this field have been made and it is expected to be a new type of promising drug for treatment of tumors in the future. Algureza proposed the concept of tumor angiogenesis in 1947 and he pointed out that, one of the important features of tumor growth is to form from the new capillary endothelial cells of the host. In 1971, Folkman proposed the hypothesis that, the tumor growth and metastasis depend on angiogenesis and believed that during the primary solid tumor period, tumor angiogenesis factors may be secreted to stimulate the host capillary proliferation. Tumor angiogenesis can not only provide tumors with the needed nutrients and oxygen and to remove the metabolites, but also can form the path of distant metastasis (Folkman, J., J. Natl. Cancer Inst.1990; 82:4-6). Therefore, blocking angiogenesis could become a means of preventing tumor growth and metastasis, and thus triggering the extensive study on the angiogenesis molecules and anti-angiogenesis molecules. Among these angiogenesis inhibitors, angiostatin and endostatin are most striking in particular, and both of which have been listed for the clinical trials in the United States, although these angiogenesis inhibitors present very attractive prospect, its flaws are also very clear: to date, the active targets of the angiogenesis drugs , such as endostatin and angiostatin, etc, are not clear, have no clear specificity and selectivity and limited effect, causing very high consumption in the test. In the animal model test of mouse, the dose of angiostatin can be up to hundreds of mg / kg body weight, and the dose of endostatin can be up to dozens of mg / kg body weight. When the angiogenesis inhibitors are used in human body, the dose should be at least a few grams / person. Such high drug use dose will surely enhance the side effect of such type of drugs in the future, causing increased drug quality control difficulty, increased production scale and production costs and high drug prices.
Thus, a good anti-angiogenic drug shall have selectivity on the tagged molecules of the neovascularization to achieve a guiding role in angiogenesis and enhance the inhibitive role of the drugs on the angiogenesis from the overall aspect: to use a very low dose of drugs to achieve high effect of inhibition of angiogenesis.

Integrin is a transmembrane protein heterodimer composed of α and β subunits. The study shows that, the integrin on the tumor cell surface is the key for tumor metastasis, which controls the cell 1 migration, differentiation and proliferation through connecting the intracellular cytoskeleton and extracellular matrix protein interaction (Schoenwaelder SM, etc., Curr Opin Cell Biol, 1999; 274-286). The majority of more than 20 types of integrins can identify the extracellular matrix ligand containing the RGD (arginine - glycine - aspartic acid) sequence (Dennis MS, etc., Proc Natl Acad Sci 1990; 87:2471-2475). The sequences containing RGD have integrin antagonist effect, can reduce the expression of cell surface adhesion molecules, regulate the intracellular signal transduction, so it has very broad application prospects in tumor treatment.

Physiochemical modification is an important process to enhance the effectiveness of polypeptides or proteins in the treatment or biotechnology. When the modified substances are bound to the protein or polypeptides in appropriate ways or the protein polypeptides are modified, they can modify many features, while the main biological activity functions, such as the enzyme activity or specific binding sites, can be retained. Physiochemical modification process can enhance the drug properties through the following means, firstly, the modified substances can be bound to the surface of proteins or polypeptide s to enhance the molecular size, to carry large amount of water molecules, and such modified substance-protein is thus increased by 5-10 times; secondly, the physiochemical modification can not only dissolve the previously insoluble proteins, but also has the feature of high degree of mobility; in addition, the modification on the protein polypeptide s can reduce the filtration of drugs through kidney, and reduce its pyrogen property, but also can reduce the digestion of protease, and enhance its transport through the protection molecules by preventing from human body's immune system attacks; meanwhile, because it avoids the human body's defense mechanism, it stays on the action site much longer, and thus enhancing the drug concentration of local parts. The PEG-polypeptide s (or PEG-protein) products currently available on the markets include many varieties, such as the new drug SD/01 developed by the world's largest biotechnology company Amgen, which is the PEG-modified granulocyte colony-stimulating factor G-CSF, and the long-acting G-CSF of the early product of Amgen; while the sales of G-CSF in 1999 was 1.22 billion USD, and 1.26 billion USD in 2000, so the market of modified protein polypeptide s products is very huge.

CN 1699408 A relates to high-efficient angiogenesis suppression polypeptide and a method for making the same.

US 6,825,167 B1 discloses a composition consisting of chimeric polypeptide comprising a peptide or polypeptide targeting moiety specific for endothelial cells linked to an endostatin polypeptide, wherein the targeting moiety is linked to the carboxy terminus of the endostatin polypeptide, and wherein the amino acid at position 125 in the endostatin polypeptide is not prolin.

U. Hersel et al., Biomaterials 24 (2003), 4385-4415, disclose a study on RGD modified polymers as biomaterials for stimulated cell adhesion.

C. Fischbach et al., Biomaterials 28 (2007), 2069-2076, disclose a study on polymers for pro- and anti-angiogenic therapy.

K. Temming et al., Drug Resistance Updates 8 (2005), 381-402 disclose RGD-based strategies for selective delivery of therapeutics and imaging agents to the tumor vasculature.

CN 1 876 186 A relates to an endostatin conjugate and its preparation method.

L. Honeycutt et al., Pharmaceutical Research, Vol. 13, No. 9, 1996, disclose a comparison of pharmacokinetic parameters of a polypeptide, the Bowman-Birk protease inhibitor (BBI), and its palmitic acid conjugate.

S. Tsunoda et al., Journal of Controlled Release 68 (2000), No. 3, 335-341, disclose a study on the molecular design of polyvinylpyrrolidone-conjugated interleukin-6 for enhancement of in vivo thrombopoietic activity in mice.

G. Gregoriadis et al., CMSL, Cell. Mol. Life Sci. 57 (2000), No. 13-14, 1964-1969, disclose a review on the potential of polysialic acids in improving the stability and pharmacokinetics of proteins and other therapeutics.

### SUMMARY OF THE INVENTION

Until so far, some small peptides encoding antiangiogenesis agent have the effect of inhibiting angiogenesis and anti-tumors. In this study, different sequences containing arginine - glycine - aspartic amino acid are added to both ends of the small polypeptides that inhibit angiogenesis to construct a kind of antiangiogenesis agent having binding effect and affinity with the integrin.

One of the objects of the present invention is to provide a kind of highly efficient antiangiogenesis agent having binding effect and affinity with the integrin. The highly efficient antiangiogenesis agent can be synthesized. The second object of the present invention is to provide a production method of said "highly efficient antiangiogenesis agent having binding effect and affinity with the integrin". The target genes can be synthesized or the product can be obtained by cloning of the target genes into the prokaryotic expression vector or a eukaryotic expression vector by PCR amplification. The third object of the invention is to provide the use of said "highly efficient antiangiogenesis agent having binding effect and affinity with the integrin" in treating tumors.

To achieve the above objects, the present invention provides an agent according to claim 1, a method according to claim 2 and a use according to claim 6. Further embodiments of the present invention are set out in the dependent claims.

The applications for use in treating tumors expressing integrin include the production of nano-drugs of all polypeptide sequences in the invention, including the production of polylactic acid (PLA) nano-particles or micro-balls, or poly-butylcyanoacrylate (PBCA) nano-particles or micro-balls, or chitosan nano-particles or micro-balls.

The endothelial cell proliferation assay, CAM analysis and in vivo anti-tumor test in mice were carried out to analyze the conditions of binding with tumor cells. These tests showed that the products in the present invention can significantly enhance and improve the effect of angiogenesis inhibitors in inhibiting endothelial cell growth and anti-tumor, with low use amount, reducing the costs, thus, the highly efficient angiogenesis inhibitor in the present invention is scientific, reasonable, feasible and effective, can be used as the medicaments for treating tumors.

The present invention can achieve the purposes as follows:
Said highly efficient angiogenesis inhibition polypeptide and its physiochemical modified products can be used to prepare the medicaments for treating the human angiogenesis-related diseases-tumors.

Compared with the similar type of products, the resulting products have highly efficient and specific inhibition on the endothelial cell proliferation and anti-tumor effects, with small dosage, reducing the side effect of medicament treatment.

In the present invention, modifications of polypeptides are carried out, which extend the half-life of polypeptides (T1 / 2), enhance the stability, reduce the immunogenicity and antigenicity, change the molecular structure and thus improve the medicament kinetic and pharmacody-namics nature, enhance the blood concentration of the affecting parts. Meanwhile, the modified polypeptides present better tolerance compared with the non-modified polypeptides, and enhance the clinical application scope and efficacy in the future.

### BRIEF DESCRIPTION OF THE DRAWINGS

FIG. 1 Results of HPLC purified RGD-ED analysis
FIG. 2 CAM analysis of RGD-ED inhibiting angiogenesis: A, blank control, B, C, D represent the treatment groups of 0.05µg, 0.1µg and 0.2µg respectively
FIG.3 RGD-ED in vivo tumor suppression effect
FIG.4 In vivo tumor suppression effect of polyethylene glycol (PEG) and liposome-modified polypeptide s: 1, 2, 3 represent the effect of inhibition of human hepatocellular carcinoma of non-modified RGD-ED and liposome-modified and PEG-modified RGD-EDs respectively

In the following embodiments/examples, those not including the polypeptide sequences and terminal modifications indicated in claim 1 are comparative embodiments/examples only.

### DETAILED DESCRIPTION OF THE PREFERRED EMBODIMENTS

### 1. RGD-ED Gene cloning and construction of prokaryotic expression vector

The bases encoding RGD-ED polypeptide sequences were synthesized as the template; and the upstream primer and downstream primer were synthesized, of which, the upstream primer was added with Ndel restriction site; while the downstream primers contained Arg-Gly-Asp sequence and Xhol site, then PCR amplification was carried out, and the amplification products were recovered through agarose gel electrophoresis and purified, then NdeI and XhoI digestion, and then cloned into prokaryotic expression vector pw, PCR screening of positive clones, and nucleotide sequence analysis confirmed that the sequence mutations have occurred in the design.
Synthetic primer 1:5 'GGAATTCCATATG ATCGTGCGCCGTGCCGACCGC3'
Synthetic primer 2:5 'CCGCTCGAGGCAGAAGCAGTCACCACGGCA3'
of which, primer 1 encoding Ndel site and part of Ile-Val-Arg-Arg-Ala-Asp-Arg-Ala-Ala-Val-Pro sequence, and the primer 2 encoding Xhol site and genes containing Arg-Gly-Asp sequences.

2. To compare the actual effect of the designed angiogenesis inhibitor RGD-ED in the present invention, in this embodiment, we entrusted the company to synthesize the polypeptide Ile-Val-Arg-Arg-Ala-Asp-Arg-Ala-Ala-Val-Pro (ED) not containing Arg-Gly-Asp sequence.

### 3. Induced expression of recombinant bacteria

The expression plasmid was used to transfected the E. coli, after the recombinant bacteria was induced to expression for 3 h by 1mMIPTG. The cells were collected and broken under ultrasound wave, and the supernatant and precipitation were centrifuged and separated, and then subject to 15% SDS-PAGE electrophoresis analysis. The SDS-PAGE stained by Coomassie brilliant blue was scanned (UVP White/Ultraviolet transilluminator) and the expression results were analyzed.

### 4. Inclusion body separation, dissolution and renaturation

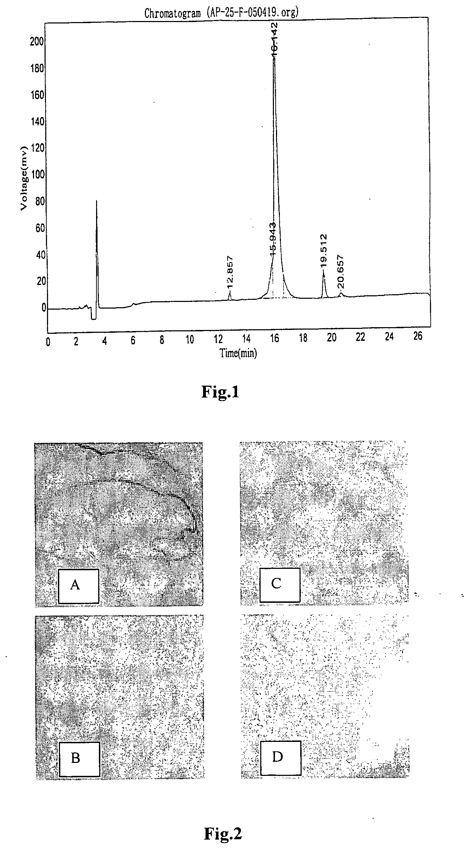

The bacteria were broken by ultrasound wave and separated through centrifugation, then the inclusion body precipitation was washed with 0.1Mtris and sodium deoxycholate. The precipitation was dissolved in the sodium Lauryl sarcosine (SLS), centrifugation for 5 min at 10000rpm under 4 °C. The supernatant was dialyzed at 4 °C with the dialysis solutions of buffer A (10mM Tris-HCl, 0.1mM oxidized glutathione and 1mM reduced glutathione, pH7.4), replaced for 3 times in total, and then dialysis for one time with the dialysis solution of buffer B (10mM Tris-HCl, pH7.4). The samples were directly SP-Sepharose Fast Flow (Amersham Pharmacia Biotech) chromatography. Buffer B solution was washed with 0.6M NaCl, Tris-HCl, pH7.4, and 1M NaCl, Tris-HCl, pH7.4, then elution step by step was carried out and the elution solution was mixed. The buffer B solution was concentrated and freeze-dried after dialysis. The chromatography results were shown in Figure 1.

### 5. Analysis of endothelial cell proliferation

The culture of BCE cells and NIH 3T3 cells, method: the culture solution DMEM contained 10% inactivated calf serum (BCS), 1% antibiotics and 3ng/ml bFGF. Cell proliferation analysis method is as follows: washed the cells with PBS, digested with trypsin, added with culture solution suspension cells and centrifuged and collected cells, and regulated the cell concentrations to 25,000 cells / ml. The cells were moved to 6-well plates (0.5ml/well) and cultured for 24h. Replaced the culture medium as 1ml DMEM, 5% BCS, 1% antibiotics, 1ng/ml bFGF. Different doses of samples were added to each hole, and further cultured for 48h, digested the cells with trypsase, re-suspended in PBS, fixed with 70% ice-cold ethanol and stained with 7-AAD, and then conducted analysis with flow cytometry.

The results showed that: Recombinant RGD-ED can specifically inhibit the endothelial cell-BCE cell proliferation, but have no inhibitory effect on non-NIH 3T3 endothelial cells. The ED₅₀ of inhibition of BCE proliferation was about 0.1µg/ml, but the polypeptide ED₅₀ without RGD sequence was about 0.8µg/ml, and the ED₅₀ of endostatin was about 0.5µg/ml. The above tests showed that, the highly-efficient angiogenesis inhibitors actually enhanced the bioactivity of the existing angiogenesis.

### 6. CAM Analysis

To detect the in vivo anti-angiogenic activity, CAM analysis was carried out. All the tests were carried out on the ultra-clean platform under sterile conditions. The 6-day disinfected chick embryos were cultured under the condition of 37°C, 90% humidity. After 2 days, the top of each egg was punched and the reagents were dripped into the sterile Whatman filter paper, then put on the CAMs vascular clustered area, cultivated for 48 hours, and observed the chick embryos and CAMs and took pictures.

As shown in Fig.2, to evaluate the RGD-ED in vivo anti-angiogenesis activity, different doses of RGD-ED were used to carry out CAM test, of which, 0.05µg, 0.1µg and 0.2µg of the RGD-ED were able to significantly inhibit the neovascularization and angiogenesis, while 0.5µgRGD-ED could completely inhibit the angiogenesis and cause chick embryo death. RGD-ED had a potential role in inhibiting angiogenesis.

7. Polypeptides of this invention can be artificially synthesized with solid or liquid phase methods.

### 8. PEG modification of polypeptide (comparative example)

### 8.1 N- terminal amino acylation modification

The polypeptide with weight percentage of about 1-70%, preferably about 20-50% was mixed with about 20% -95% of the polyethylene glycol SC-mPEG (with an average molecular weight of 5000) or SS-PEG (succinamide -type) or PEG - isocyanate solution, preferably about 50-93% of the above-mentioned modified substance, mixed fully and shaken on the shaker at temperature of 4 °C -40 °C, preferably 25 °C -37 °C for more than 10 min, and then the N-terminal modified products were separated through ion-exchange chromatography.

### 8.2 Carboxy-terminal modification

The polypeptides with weight percentage of about 1-70%, preferably about 20-50% were fully mixed with about 20% -95% mPEG-NH2 (average molecular weight of 5000) and small amount of DCCI (dicyclohexyl carbodiimide) solution, preferably about 50-93% of the above modified substances, and then shaken at the shaker at 4 °C -40 °C, preferably 22 °C -37 °C for more than 10 min, preferably 90min. The carboxyl group could bind with the amino-group of mPEG-NH2 to form amide bond, and then N-terminal modified product was obtained through RP-HPLC separation.

### 8.3 Thiol-terminal Modification

The polypeptides with weight percentage of about 1-70%, preferably about 1-30% were fully mixed with about 20% -95% mPEG-MAL (average molecular weight of 5000) solution. Preferably about 70 - 94% of the above modified substance was fully mixed even, and shaken on the shaker at 4 °C - 40 °C, preferably 20°C - 37 °C for more than 10 min, preferably 10min, and then N-terminal modified products were obtained through ion-exchange separation.

### 9. Other modification methods

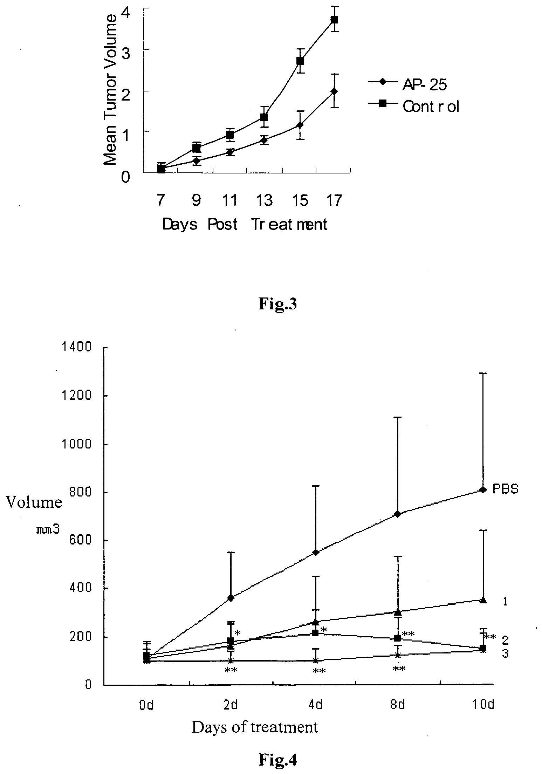

Other modification methods refer to PEG modification, of which, the PEG-modification and liposome-modification (comparative example) have the best modification effects (see Fig. 4).

### 10. In Vivo Anti-tumor Test

The cultured B16F10 melanoma cells were treated with 0.05% trypsin, centrifuged at 1000 rpm for 5 min, suspended again in PBS, inoculated subcutaneously with 0.1ml of 5×10⁵ cells at the body sides of C57BL/6 mice (6-8 weeks). When the average tumor size reached 200mm³-300 mm³, the mice were randomly divided into two groups, 7 mice for each group, of which, one group of mice were treated with RGD-ED, and the other group were treated with ED not containing RGD, with the doses of 5mg/kg / d for the two groups, and the control group of mice were subject to PBS injection. The treatment method was contralateral subcutaneous injection of the inoculated tumors. Every day, the tumor size was measured with a vernier caliper to calculate the tumor volume with the formula: tumor volume = length × width² × 0.52, and the treatment efficacy was represented with the tumor inhibition rate within the given period of time: (1 - T / C) × 100%, T =tumor volume of treatment group, C = tumor volume of control group.

As shown in Fig.3, the results showed that, on the 9^{th} day, the RGD-ED tumor inhibition rate was 58%, while the tumor inhibition rate of polypeptide ED not containing RGD sequence was 28%. The above tests showed that, the highly efficient angiogenesis inhibitors of this invention could significantly inhibit the tumor growth in the mouse body.

The cultured human liver cancer SGC7901 was treated with 0.05% trypsin, centrifuged at 1000 rpm for 5 min, suspended again in PBS, inoculated subcutaneously with 0.1ml of 5×10⁵ cells on the body sides of nude mice (6-8 weeks). When the average tumor size reached 100mm³-200 mm³, the mice were randomly divided into three groups, 7 mice for each group, of which, one group of mice were treated with RGD-ED, one group were treated with RGD-ED modified with liposome, and the third group were treated with RGD-ED modified with polyethylene glycol, with the doses of 3mg/kg / d for the three groups, and the control group of mice were subject to PBS injection. The treatment method was contralateral subcutaneous injection of the inoculated tumors. Every day, the tumor size was measured with a vernier caliper to calculate the tumor volume according to the formula: tumor volume = length × width² × 0.52, and the treatment efficacy was represented by the tumor inhibition rate within the given period of time: (1 - T / C) × 100%, T =tumor volume of treatment group, C = tumor volume of control group.

As shown in Fig.4. the results showed that, on the 10^{th} day, the RGD-ED tumor inhibition rate was 68%, the liposome-modified RGD-ED tumor inhibition rate was 72%, and the polyethylene glycol-modified RGD - ED tumor inhibition rate was 78%. The above test showed that, the modification products obtained according to the designed modification method could significantly inhibit the tumor growth in the mouse body.
Example 2: The procedure was carried out basically as Example 1, but, wherein the gene sequence:
   Arg-Gly-Asp containing Primer 2 encoding XhoI site should be replaced by the sequence Arg-Gly-Asp-Gly-Gly-Gly-Gly, and then the amplified Ile-Val-Arg-Arg-Ala-Asp-Arg-Ala-Ala-Val-Pro-Gly-Gly-Gly-Gly-Arg-Gly-Asp sequence was cloned.
Example 3 The procedure was carried out basically as Example 1, but, the full-length gene synthesis of gene sequence of Arg-Gly-Asp-Gly-Gly-Gly-Gly-Ile-Val-Arg-Arg-Ala-Asp- Arg-Ala-Ala-Val-Pro, and then cloned.
Example 4 The procedure was carried out basically as Example 1, but, the full-length gene synthesis of gene sequence of Arg-Gly-Asp-Ile-Val-Arg-Arg-Ala-Asp-Arg-Ala-Ala-Val-Pro, and then cloned.
Example 5 The procedure was carried out basically as Example 1, but, the full-length gene synthesis of gene sequence of Arg-Gly-Asp-Ile-Val-Arg-Arg-Ala-Asp-Arg-Ala-Ala-Val-Pro-Arg-Gly-Asp, and then cloned.
Example 6 The procedure was carried out basically as Example 1, but, the full-length gene synthesis of gene sequence of Arg-Gly-Asp-Gly-Gly-Gly-Gly-Ile-Val-Arg-Arg-Ala-Asp- Arg-Ala-Ala-Val-Pro-Gly-Gly-Gly-Gly- Arg-Gly-Asp, and then cloned.
Example 7 The procedure was carried out basically as Example 1, but, the full-length gene synthesis of gene sequence of Arg-Gly-Asp-Ile-Val-Arg-Arg-Ala-Asp-Arg-Ala-Ala- Val-Pro-Gly-Gly-Gly-Gly- Arg-Gly-Asp, and then cloned.
Example 8 The procedure was carried out basically as Example 1, but, the full-length gene synthesis of gene sequence of Arg-Gly-Asp-Gly-Gly-Gly-Gly-Ile-Val-Arg-Arg-Ala-Asp-Arg-Ala-Ala-Val-Pro-Arg-Gly-Asp, and then cloned.
Example 9 The procedure was carried out basically as Example 1, but, the full-length gene synthesis of gene sequence of Ala-Cys-Asp-Cys-Arg-Gly-Asp-Cys-Phe-Cys-Gly-Gly-Gly-Gly-Ile-Val-Arg-Arg-Ala-Asp-Arg-Ala-Ala-Val-Pro, and then cloned.
Example 10 The procedure was carried out basically as Example 1, but, the full-length gene synthesis of gene sequence of Ala-Cys-Asp-Cys-Arg-Gly-Asp-Cys-Phe-Cys-Ile-Val-Arg-Arg-Ala-Asp-Arg-Ala-Ala-Val-Pro, and then cloned.
Example 11 The procedure was carried out basically as Example 1, but, the full-length gene synthesis of gene sequence of Ile-Val-Arg-Arg-Ala-Asp-Arg-Ala-Ala-Val-Pro-Gly-Gly- Gly-Gly-Ala-Cys-Asp-Cys-Arg-Gly-Asp-Cys-Phe -Cys, and then cloned.
Example 12 The procedure was carried out basically as Example 1, but, the full-length gene synthesis of gene sequence of Ile-Val-Arg-Arg-Ala-Asp-Arg-Ala-Ala-Val-Pro-Ala-Cys-Asp-Cys-Arg-Gly-Asp-Cys-Phe-Cys, and then cloned.
Example 13 PEG modification of polypeptides (comparative example)

### 13.1 N- terminal amino acylation modification

Polypeptides with weight percentage of about 1-70%, preferably about 20-50% was mixed with about 20% -95% of polyethylene glycol SC-mPEG (with an average molecular weight of 5000) or SS-PEG (succinamides) or PEG - isocyanate solution, preferably about 50-93% of the above-mentioned modified substance, then fully mixed even, and shaken on a shaker at temperature of 4 °C -40°C, preferably 25 °C -37 °C for more than 10 min, and then the N-terminal modified products were separated through ion-exchange chromatography.

### 13.2 Carboxy-terminal modification

Polypeptides with weight percentage of about 1-70%, preferably about 20-50% were fully mixed with about 20% -95% mPEG-NH2 (average molecular weight of 5000) and small amount of DCCI (dicyclohexyl carbodiimide) solution, preferably about 50-93% of the above modified substances were fully mixed even, and then shaken at a shaker at 4 °C -40 °C, preferably 22 °C -37 °C for more than 10 min, preferably 90min. The carboxyl group could bind with the amino-group of mPEG-NH2 to form amide bond, and then N-terminal modified products were obtained through RP-HPLC separation.

### 13.3 Thiol-terminal Modification

Polypeptides with weight percentage of about 1-70%, preferably about 1-30% were fully mixed with about 20% -95% mPEG-MAL (average molecular weight of 5000) solution. Preferably about 70 - 94% of the above modified substance was fully mixed even, and shaken on a shaker at 4 °C -40 °C, preferably 20°C -37°C for more than 10 min, preferably 60 min, and then N-terminal modified products were obtained through ion-exchange separation.

### 13.4 In Vivo Anti-tumor Test

The cultured human liver cancer SGC7901 was treated with 0.05% trypsin, centrifuged at 1000 rpm for 5 min, suspended again in PBS, inoculated subcutaneously with 0.1ml of 5×10⁵ cells on the body sides of nude mice (6-8 weeks). When the average tumor size reached 100mm³-200 mm³, the mice were randomly divided into three groups, 7 mice for each group, of which, one group of mice were treated with RGD-ED, one group were treated with liposome-modified RGD-ED, and the third group were treated with polyethylene glycol-modified RGD-ED, with the doses of 3mg/kg / d for the three groups, and the control group of mice were subject to PBS injection. The treatment method was contralateral subcutaneous injection of the inoculated tumors. Every day, the tumor size was measured with a vernier caliper to calculate the tumor volume according to the formula: tumor volume = length × width² × 0.52, and the treatment efficacy was represented by the tumor inhibition rate within the given period of time: (1 - T / C) × 100%, T =tumor volume of treatment group, C = tumor volume of control group.

As shown in Fig.4, the results showed that, on the 10^{th} day, the RGD-ED tumor inhibition rate was 68%, the liposome-modified RGD-ED tumor inhibition rate was 72%, and the polyethylene glycol-modified RGD - ED tumor inhibition rate was 78%. The above test showed that, the modification products obtained according to the designed modification method could significantly inhibit the growth of tumors in the mouse body.

Example 14 The procedure was carried out basically as Example 13, but wherein low molecular weight heparin modification was adopted.

Polypeptides with weight percentage of about 10% -90%, preferably about 25% -50% were mixed with about 10% -90% activated low molecular weight of heparin, preferably about 43% -75%, and slightly shaken in buffer solution of pH 7-9 at 4 °C for more than 18h, and then the free amino- modified products were separated through ion-exchange chromatography method.

Example 15 The procedure was carried out basically as Example 13, but, wherein PEG-PLA modification was adopted (comparative example)

80mg-120mg of PEG-PLA and 15mg-30mg polypeptide s were dissolved in 40ml of dimethylformamide (DMF) and the resulting mixture was transferred to dialysis bag with MWCO of 3500, and dialyzed for 48 h in 3L-4L of distilled water, removed of precipitation through centrifugation. The supernatant was filtered through 0.45µm filter membrane to obtain the polymer micellar solution.

Example 16 The procedure was carried out basically as Example 13, but, wherein dextran modification was adopted (comparative example).

lg dextran (molecular weight of 35,000) was activated and added with 10mg-60mg of polypeptide, preferably 20mg-45mg, slowly shaken on a shaker at 4 °C for more than 15h, and then the free amino- modified products were separated through ion-exchange chromatography method.

Example 17 The procedure was carried out basically as Example 13, but, wherein palmitic acid modification was adopted.

Polypeptides with weight percentage of about 5%-90%, preferably about 25% -60% were mixed with about 10% -95% activated palmitic acid, preferably about 43% -90%, and shaken in a shaker at 25°C -37°C for reactions for more than 30 min, and then the free amino- modified products were separated through ion-exchange chromatography method.

Example 18 The procedure was carried out basically as Example 13, but, wherein colominic acid modification was adopted.

Polypeptides with weight percentage of about 1%-90%, preferably about 7%-50% were mixed with about 10% -95% activated colominic acid, preferably about 50%-93%, and shaken in a shaker at 4°C -40°C preferably 25°C -37°C for reactions for more than 15h, and then the free amino- modified products were separated through ion-exchange chromatography method.

Example 19 The procedure was carried out basically as Example 13, but, wherein liposomes modification was adopted, including REV, DRV and Mvl (comparative example).

A certain percentage of soybean lecithin and cholesterol were dissolved in chloroform, and evaporated into thin film at 35 °C -45 °C under reduced pressure condition, and then dissolved in anhydrous ether. 6mg-10mg polypeptide s were weighed and dissolved in 6ml pH6-8 phosphate buffer. The buffer solution was mixed with the phospholipid solution, shaken for 4min-9min under ultrasonic condition, and then evaporated to remove organic solvent at 20 °C -30 °C under reduced pressure condition, and then dried under vacuum conditions at 60 °C, and then immersed with phosphate buffer solution for 4h to obtain the liposome suspension. The suspension was circulated for several times through a high-pressure homogenization machine to obtain the liposomes.

Example 20 The procedure was carried out basically as Example 13, but, wherein nano-drug preparation was adopted, including PLA, PBCA and chitosan nanoparticles (comparative example).

10mg-50mg of chitosan was mixed with 20ml-40ml of water, and under ultrasonic condition for 20-40min, to obtain micro-sphere dispersions. The added 3.5mg-6mg polypeptide s were dissolved in 2ml-5ml of anhydrous methanol, then slowly dripped with micro-sphere dispersion during the ultrasonic process to encapsulate the polypeptides, and then centrifuged to obtain nano-particles.

Example 21 Analysis of the binding of polypeptide s and integrin by flow cytometry. Bel-7402 tumor cells were used, which can express integrin. The cells were cultured in a 24-well plate, and the cells were collected and washed with ice-cold PBS twice. Before labeling, the cells were suspended in PBS +1% BSA, maintained for 30min, added with 1 µl FITC labeled polypeptide s (P1, P2, P3, P4, P5, P6, P7, P8, P9, P10, P11, P12, P13, P14, P15, P16, RGD and ES-2) for reaction for 25 min. After labeling, the cells were collected and washed twice with PBS, suspended again in 400 µl PBS and analysis was conducted with a flow cytometry (Becton Dickinson, USA). FITC fluorescence was determined at FL1 channel. The cells with FITC feature would be further analyzed. The results showed that, after modification, adding of polypeptide containing RGD sequences can specifically bind with the tumor cells expressing integrin (Table 1).

**Table 1 Results of tumor targeting analysis after adding sequences containing RGD sequences**

| Group | Dosage (µg/µl) | Binding Value |
|---|---|---|
| Negative control group(ES-2) | 1 | 18.35 |
| P1 | 1 | 63.21 |
| P2 | 1 | 62.15 |
| P3 | 1 | 58.96 |
| P4 | 1 | 60.13 |
| P5 | 1 | 59.40 |
| P6 | 1 | 60.27 |
| P7 | 1 | 60.25 |
| P8 | 1 | 58.30 |
| P9* | 1 | 66.04 |
| P10 | 1 | 63.28 |
| P11 | 1 | 60.20 |
| P12* | 1 | 67.28 |
| P13 | 1 | 59.25 |
| P14 | 1 | 58.32 |
| P15 | 1 | 64.11 |
| P16* | 1 | 66.12 |
| RGD | 1 | 59.45 |

| | | |
|---|---|---|
| * according to the present invention | | |

Example 22 Survey on the half-life of polypeptides in plasma at 37 °C after modification. Preparation of blood drug samples: after crude drug (volume V) (800µg/ml) +1V plasma were mixed and diluted with 2V water evenly, incubated for 0,5,30 min at 37 °C respectively, immediately heated at 80 °C for 30 min; the negative control: after 1V plasma were diluted and mixed with 3 V water evenly, and heated for 30 min at 80 °C. The positive control group: after 1V crude drug (800µg/ml) were diluted and mixed evenly with 3V water, heated for 30 min at 80°C. The samples were the modified products, heated at 14000rpm for 10min. The supernatant was fetched for HPLC analysis with the injection size of 20µl. The results showed that, the half-life of polypeptides after modification significantly increased (See Table 2).

**Table 2 Analysis of anti-tumor effect of polypeptides after modification**

| **Group** | **Dosage (800µg/ml)** | **Half life of plasma(min)** |
|---|---|---|
| Crude drug P1 | 20µl | 7 |
| Polyethylene glycol-modified substance* | 20µl | 18 |
| Low molecular weight heparin-modified substance | 20µl | 16 |
| Dextran-modified substance* | 20µl | 15 |
| polyvinyl pyrrolidone-modified substance | 20µl | 24 |
| Polyethylene glycol - poly-amino acid copolymer modified substance* | 20µl | 32 |
| Palmitic acid-modified substance | 20µl | 12 |
| Polysialic acid modified substance | 20µl | 15 |
| Liposome modified substance* | 20µl | 21 |
| Preparation of P1 polypeptide with nano-particles* | 20µl | 66 |

| | | |
|---|---|---|
| * comparative example | | |

### SEQUENCE LISTING

<110> Applicant: Hanmei, XU
<120> Title: AN INHIBITING AGENT FOR INHIBITION OF ANGIOGENESIS, A METHOD FOR PREPARING THE AGENT, A METHOD FOR MODIFYING THE AGENT AND ITS USE FOR MANUFACTURING A MEDICAMENT FOR TREATING TUMOR
<160> Number of the sequences in the sequence listing: 12
<210> Sequence identifier corresponding to the sequence: 1
   <211> Sequence length: 25 amino acids
   <212> Sequence type: PRT
   <213> Organism: Artificial sequence
<400> Sequence identifier: 
Ala-Cys-Asp-Cys-Arg-Gly-Asp-Cys-Phe-Cys-Gly-Gly-Gly-Gly-Ile-Val-Arg-Arg-Ala
3 6 9 12 15 18
-Asp-Arg-Ala-Ala-Val-Pro
21 24
<210> Sequence identifier corresponding to the sequence: 2
   <211> Sequence length: 21 amino acids
   <212> Sequence type: PRT
   <213> Organism: Artificial sequence
<400> Sequence identifier: 
Ala-Cys-Asp-Cys-Arg-Gly-Asp-Cys-Phe-Cys-Ile-Val-Arg-Arg-Ala-Asp-Arg-Ala-Ala-Val-Pro
3 6 9 12 15 18 21
<210> Sequence identifier corresponding to the sequence: 3
   <211> Sequence length: 18 amino acids
   <212> Sequence type: PRT
   <213> Organism: Artificial sequence
<400> Sequence identifier: 
Arg-Gly-Asp-Gly-Gly-Gly-Gly-Ile-Val-Arg-Arg-Ala-Asp-Arg-Ala-Ala-Val-Pro
3 6 9 12 15 18
<210> Sequence identifier corresponding to the sequence: 4
   <211> Sequence length: 14 amino acids
   <212> Sequence type: PRT
   <213> Organism: Artificial sequence
<400> Sequence identifier: 
Arg-Gly-Asp-Ile-Val-Arg-Arg-Ala-Asp-Arg-Ala-Ala-Val-Pro
3 6 9 12
<210> Sequence identifier corresponding to the sequence: 5
   <211> Sequence length: 25 amino acids
   <212> Sequence type: PRT
   <213> Organism: Artificial sequence
<400> Sequence identifier: 
Ile-Val-Arg-Arg-Ala-Asp-Arg-Ala-Ala-Val-Pro-Gly-Gly-Gly-Gly-
3 6 9 12 15
Ala-Cys-Asp-Cys-Arg-Gly-Asp-Cys-Phe-Cys
18 21 24
<210> Sequence identifier corresponding to the sequence: 6
   <211> Sequence length: 21 amino acids
   <212> Sequence type: PRT
   <213> Organism: Artificial sequence
<400> Sequence identifier: 
Ile-Val-Arg-Arg-Ala-Asp-Arg-Ala-Ala-Val-Pro-Ala-Cys-Asp-Cys-Arg-Gly-Asp-Cys- Phe-Cys
3 6 9 12 15 18 21
<210> Sequence identifier corresponding to the sequence: 7
   <211> Sequence length: 18 amino acids
   <212> Sequence type: PRT
   <213> Organism: Artificial sequence
<400> Sequence identifier: 
Ile-Val-Arg-Arg-Ala-Asp-Arg-Ala-Ala-Val-Pro-Gly-Gly-Gly-Gly- Arg-Gly-Asp
3 6 9 12 15 18
<210> Sequence identifier corresponding to the sequence: 8
   <211> Sequence length: 14 amino acids
   <212> Sequence type: PRT
   <213> Organism: Artificial sequence
<400> Sequence identifier: 
Ile-Val-Arg-Arg-Ala-Asp-Arg-Ala-Ala-Val-Pro- Arg-Gly-Asp
3 6 9 12
<210> Sequence identifier corresponding to the sequence: 9
   <211> Sequence length: 17 amino acids
   <212> Sequence type: PRT
   <213> Organism: Artificial sequence
<400> Sequence identifier: 
Arg-Gly-Asp-Ile-Val-Arg-Arg-Ala-Asp-Arg-Ala-Ala-Val-Pro- Arg-Gly-Asp
3 6 9 12 15
<210> Sequence identifier corresponding to the sequence: 10
   <211> Sequence length: 25 amino acids
   <212> Sequence type: PRT
   <213> Organism: Artificial sequence
<400> Sequence identifier: 
Arg-Gly-Asp-Gly-Gly-Gly-Gly-Ile-Val-Arg-Arg-Ala-Asp-Arg-Ala-Ala-Val-Pro-Gly-
3 6 9 12 15 18
Gly-Gly-Gly- Arg-Gly-Asp
21 24
<210> Sequence identifier corresponding to the sequence: 11
   <211> Sequence length: 21 amino acids
   <212> Sequence type: PRT
   <213> Organism: Artificial sequence
<400> Sequence identifier: 
Arg-Gly-Asp-Ile-Val-Arg-Arg-Ala-Asp-Arg-Ala-Ala-Val-Pro-Gly-Gly-Gly-Gly-
3 6 9 12 15 18
Arg-Gly-Asp
21
<210> Sequence identifier corresponding to the sequence: 12
   <211> Sequence length: 21 amino acids
   <212> Sequence type: PRT
   <213> Organism: Artificial sequence
<400> Sequence identifier:

Arg-Gly-Asp-Gly-Gly-Gly-Gly-Ile-Val-Arg-Arg-Ala-Asp-Arg-Ala-Ala-Val-Pro-Arg- Gly-Asp
3 6 9 12 15 18 21

## Claims

1. A highly efficient antiangiogenesis agent, which is a polypeptide selected from the group consisting of 
Ala-Cys-Asp-Cys-Arg-Gly-Asp-Cys-Phe-Cys-Ile-Val-Arg-Arg-Ala-Asp-Arg-Ala-Ala-Val-Pro-
Ala-Cys-Asp-Cys-Arg-Gly-Asp-Cys-Phe-Cys,
Ala-Cys-Asp-Cys-Arg-Gly-Asp-Cys-Phe-Cys-linker-Ile-Val-Arg-Arg-Ala-Asp-Arg-Ala-Ala-
Val-Pro-Ala-Cys-Asp-Cys-Arg-Gly-Asp-Cys-Phe-Cys,
Ala-Cys-Asp-Cys-Arg-Gly-Asp-Cys-Phe-Cys-Ile-Val-Arg-Arg-Ala-Asp-Arg-Ala-Ala-Val-Pro-
linker-Ala-Cys-Asp-Cys-Arg-Gly-Asp-Cys-Phe-Cys, and
Ala-Cys-Asp-Cys-Arg-Gly-Asp-Cys-Phe-Cys-linker-Ile-Val-Arg-Arg-Ala-Asp-Arg-Ala-Ala-
Val-Pro-linker-Ala-Cys-Asp-Cys-Arg-Gly-Asp-Cys-Phe-Cys,
 terminally modified by low molecular weight heparin, or polyvinylpyrrolidone, or palmitic acid, or colominic acid, or polysialic acid.

2. A production method of a highly efficient antiangiogenesis agent as claimed in Claim 1, wherein the procedures are as follows:
providing a polypeptide indicated in claim 1, and
implementing low molecular weight heparin modification of said polypeptide; or
implementing polyvinylpyrrolidone (PVP) modification of said polypeptide; or
implementing palmitic acid modification of said polypeptide; or
implementing colominic acid modification of said polypeptide; or
implementing polysialic acid modification of said polypeptide.

3. The method according to Claim 2, wherein said polypeptides and base sequences encoding said polypeptides can be formed through chemical synthesis method.

4. The method according to Claim 2, wherein said polypeptides and base sequences encoding said polypeptides can be formed through the following genetic engineering methods:
Synthesize the angiogenesis inhibition gene sequences containing Arg-Gly-Asp (RGD) integrin-binding sequence polypeptide segments and the polypeptide segment Ile-Val-Arg-Arg-Ala-Asp-Arg-Ala-Ala-Val-Pro (ED), and use this sequence as a template to design the upstream and downstream primers, and supplement the appropriate cloning restriction sites on the 5 'end and 3' end, to obtain RGD-ED gene by PCR amplification. The genes are cloned in the vector to screen the positive clones and carry out nucleotide sequence analysis and identification. Recombination of RGD-ED gene and prokaryotic expression vector forms the expression plasmid to transform into E. coli, IPTG-induced expression of RGD-ED and the expression products exist in the form of inclusion body;
Carry out inclusion body protein separation, dissolution and renaturation, and conduct ion-exchange chromatography for separation and purification of RGD-ED protein products and collect filtration liquid, and then frozen-drying.
Recombination of all RGD-ED genes and eukaryotic expression vector forms the expression plasmid and transform into eukaryotic cells, to induce the expression of RGD-ED, and then the expression products are isolated and purified.

5. The method according to Claim 4, wherein said polypeptide modification steps are:
(1) Polypeptides with weight percentage of 1-70% are mixed with 20%-95% low molecular weight heparin, or polyvinylpyrrolidone, or palmitic acid, or colominic acid, or polysialic acid;
(2) shaken at a shaker for more than 10min at 4 °C -40 °C, preferably at 25 °C -37 °C;
(3) The terminally modified products are separated.

6. The highly efficient antiangiogenesis agent according to Claim 1 for use in treating tumors.

## Patentansprüche

1. Hocheffizientes Antiangiogenesemittel, das ein Polypeptid ist, das aus der Gruppe, bestehend aus 
Ala-Cys-Asp-Cys-Arg-Gly-Asp-Cys-Phe-Cys-Ile-Val-Arg-Arg-Ala-Asp-Arg-Ala-Ala-Val-Pro-
Ala-Cys-Asp-Cys-Arg-Gly-Asp-Cys-Phe-Cys,
Ala-Cys-Asp-Cys-Arg-Gly-Asp-Cys-Phe-Cys-Linker-Ile-Val-Arg-Arg-Ala-Asp-Arg-Ala-Ala-Val-
Pro-Ala-Cys-Asp-Cys-Arg-Gly-Asp-Cys-Phe-Cys,
Ala-Cys-Asp-Cys-Arg-Gly-Asp-Cys-Phe-Cys-Ile-Val-Arg-Arg-Ala-Asp-Arg-Ala-Ala-Val-Pro-
Linker-Ala-Cys-Asp-Cys-Arg-Gly-Asp-Cys-Phe-Cys und
Ala-Cys-Asp-Cys-Arg-Gly-Asp-Cys-Phe-Cys-Linker-Ile-Val-Arg-Arg-Ala-Asp-Arg-Ala-Ala-Val-
Pro-Linker-Ala-Cys-Asp-Cys-Arg-Gly-Asp-Cys-Phe-Cys,
 endständig modifiziert durch Heparin mit niedrigem Molekulargewicht oder Polyvinylpyrrolidon oder Palmitinsäure oder Colominsäure oder Polysialinsäure, ausgewählt ist.

2. Verfahren zur Herstellung eines hocheffizienten Antiangiogenesemittels nach Anspruch 1, wobei die Vorgänge wie folgt sind:
Bereitstellen eines Polypeptids nach Anspruch 1 und
Durchführen einer Modifizierung des Polypeptids mit Heparin mit niedrigem Molekulargewicht; oder
Durchführen einer Polyvinylpyrrolidon (PVP)-Modifizierung des Polypeptids; oder
Durchführen einer Palmitinsäure-Modifizierung des Polypeptids; oder
Durchführen einer Colominsäure-Modifizierung des Polypeptids; oder
Durchführen einer Polysialid-Modifizierung des Polypeptids.

3. Verfahren nach Anspruch 2, bei dem die Polypeptide und Basensequenzen, welche die Polypeptide kodieren, durch ein chemisches Syntheseverfahren gebildet werden können.

4. Verfahren nach Anspruch 2, bei dem die Polypeptide und Basensequenzen, welche die Polypeptide kodieren, durch die folgenden gentechnischen Verfahren hergestellt werden können:
Synthetisieren der Angiogenesehemmungsgensequenzen, die Arg-Gly-Asp (RGD)-Integrinbindende Sequenz-Polypeptidsegmente und das Polypeptidsegment Ile-Val-Arg-Arg-Ala-Asp-Arg-Ala-Ala-Val-Pro (ED) enthalten, und Verwenden dieser Sequenz als Templat zum Gestalten der stromaufwärtigen und stromabwärtigen Primer, und Ergänzen der geeigneten Klonierungsrestriktionsstellen an dem 5'-Ende und dem 3'-Ende, so dass das RGD-ED-Gen durch PCR-Amplifikation erhalten wird. Die Gene werden in den Vektor zum Screenen der positiven Klone und zum Durchführen einer Nukleotidsequenzanalyse und -identifizierung geklont.
Eine Rekombination des RGD-ED-Gens und eines prokaryotischen Expressionsvektors bildet das Expressionsplasmid zum Transformieren in E. coli, eine IPTG-induzierte Expression von RGD-ED und die Expressionsprodukte liegen in der Form eines Einschlusskörpers vor;
Durchführen eines Trennens, Lösen und Renaturierens eines Einschlusskörperproteins und Durchführen einer lonenaustauschchromatographie zum Trennen und Reinigen von RGD-ED-Proteinprodukten und Sammeln einer Filtrationsflüssigkeit, und dann Gefriertrocknen.
Eine Rekombination aller RGD-ED-Gene und eines eukaryotischen Expressionsvektors bildet das Expressionsplasmid und eine Transformation in eukaryotische Zellen wird durchgeführt, so dass die Expression von RGD-ED induziert wird, und dann werden die Expressionsprodukte isoliert und gereinigt.

5. Verfahren nach Anspruch 4, bei dem die Polypeptid-Modifizierungsschritte sind:
(1) Polypeptide mit einem Gewichtsprozentsatz von 1 - 70 % werden mit 20 % - 95 % Heparin mit niedrigem Molekulargewicht oder Polyvinylpyrrolidon oder Palmitinsäure oder Colominsäure oder Polysialinsäure gemischt;
(2) Schütteln in einem Schüttler für mehr als 10 min bei 4 °C - 40 °C, vorzugsweise bei 25 °C - 37 °C;
(3) die endständig modifizierten Produkte werden getrennt.

6. Hocheffizientes Antiangiogenesemittel nach Anspruch 1 zur Verwendung bei der Behandlung von Tumoren.

## Revendications

1. Agent anti-angiogenèse hautement efficace, qui est un polypeptide choisi dans le groupe constitué par 
Ala-Cys-Asp-Cys-Arg-Gly-Asp-Cys-Phe-Cys-Ile-Val-Arg-Arg-Ala-Asp-Arg-Ala-Ala-Val-Pro-Ala-Cys-
Asp-Cys-Arg-Gly-Asp-Cys-Phe-Cys,
Ala-Cys-Asp-Cys-Arg-Gly-Asp-Cys-Phe-Cys-lieur-Ile-Val-Arg-Arg-Ala-Asp-Arg-Ala-Ala-Val-Pro-Ala-
Cys-Asp-Cys-Arg-Gly-Asp-Cys-Phe-Cys,
Ala-Cys-Asp-Cys-Arg-Gly-Asp-Cys-Phe-Cys-Ile-Val-Arg-Arg-Ala-Asp-Arg-Ala-Ala-Val-Pro-lieur-Ala-
Cys-Asp-Cys-Arg-Gly-Asp-Cys-Phe-Cys, et
Ala-Cys-Asp-Cys-Arg-Gly-Asp-Cys-Phe-Cys-lieur-Ile-Val-Arg-Arg-Ala-Asp-Arg-Ala-Ala-Val-Pro-lieur-
Ala-Cys-Asp-Cys-Arg-Gly-Asp-Cys-Phe-Cys,
 modifié en phase terminale par de l'héparine de bas poids moléculaire ou de la polyvinylpyrrolidone ou de l'acide palmitique ou de l'acide colominique ou de l'acide polysialique.

2. Procédé de production d'un agent anti-angiogenèse hautement efficace selon la revendication 1, dans lequel les modes opératoires sont les suivants :
fournir un polypeptide indiqué dans la revendication 1, et
mettre en oeuvre une modification d'héparine de bas poids moléculaire dudit polypeptide ; ou
mettre en oeuvre une modification de polyvinylpyrrolidone (PVP) dudit polypeptide ; ou
mettre en oeuvre une modification de l'acide palmitique dudit polypeptide ; ou
mettre en oeuvre une modification de l'acide colominique dudit polypeptide; ou
mettre en oeuvre une modification de l'acide polysialique dudit polypeptide.

3. Procédé selon la revendication 2, dans lequel lesdits polypeptides et séquences de bases codant lesdits polypeptides peuvent être formés par un procédé de synthèse chimique.

4. Procédé selon la revendication 2, dans lequel lesdits polypeptides et séquences de bases codant lesdits polypeptides peuvent être formés par les procédés de génie génétique suivants :
Synthétiser les séquences du gène d'inhibition d'angiogenèse contenant les segments polypeptidique de séquence de liaison à l'intégrine Arg-Gly-Asp (RGD) et le segment polypeptidique Ile-Val-Arg-Arg-Ala-Asp-Arg-Ala-Ala-Val-Pro (ED), et utiliser cette séquence comme une matrice pour concevoir les amorces en amont et en aval, et compléter les sites de restriction de clonage approprié à l'extrémité 5' et à l'extrémité 3', pour obtenir un gène RGD-ED par amplification PCR. Les gènes sont clonés dans le vecteur pour filtrer les clones positifs et effectuer des analyses et une identification de séquence nucléotidique.
Une recombinaison du gène RGD-ED et du vecteur d'expression procaryote forme l'expression plasmide pour se transformer en E. coli, l'expression induite par IPTG de RGD-ED et l'expression de produits existent sous la forme d'un corps d'inclusion ;
Effectuer une séparation des protéines du corps d'inclusion, une dissolution et une renaturation, et conduire une chromatographie d'échange d'ions pour une séparation et une purification des produits protéiques RGD-ED et collecter un liquide de filtration, puis un séchage par congélation. Une recombinaison de tous les gènes RGD-ED et un vecteur d'expression eucaryote forme l'expression plasmide et se transforme en cellules eucaryotes, pour induire l'expression de RGD-ED, puis les expressions de produits sont isolés et purifiés.

5. Procédé selon la revendication 4, dans lequel lesdites étapes de modification polypeptidique sont :
(1) Des polypeptides avec un pourcentage en poids de 1-70% sont mélangés avec 20-95% d'héparine de bas poids moléculaire, ou de polyvinylpyrrolidone, ou d'acide palmitique, ou d'acide colominique, ou d'acide polysialique ;
(2) agité au shaker pendant plus de 10 minutes à 4 °C - 40 °C, de préférence à 25 °C - 37° C ;
(3) Les produits modifiés en phase terminale sont séparés.

6. Agent anti-angiogenèse hautement efficace selon la revendication 1 pour utilisation dans le traitement de tumeurs.
